# EUROPEAN PATENT APPLICATION

(11) **EP 2 524 690 A1**
(43) Date of publication of application: **21.11.2012**
(21) Application number: 11004059.9
(22) Date of filing: 17.05.2011
(51) Int. Cl.: A61K 9/51

(54) **Method for making customised nanoparticles, nanoparticles and use thereof**

(71) Applicant: ETH Zurich, 8092 Zürich (CH)
(72) Inventor: Moscatelli, Davide, 8050 Zürich (CH); Lattuada, Marco, 8046 Zürich (CH); Morbidelli, Massimo, 8046 Zürich (CH); Yu, Yingchuan, 116021 Dalian (CN)

(57) **Abstract**

The invention proposes a method for the production of biodegradable nanoparticles with an average particle size of less than 400 nm. According to the invention, in a first step a macromonomer is prepared in a ring opening polymerization process between a hydrophilic acrylate compound (A) as an initiator and hydrophobic cyclic monomers (B), wherein the macromonomer comprises at least two repetitive units based on the cyclic monomer. In a second step this macromonomer or a mixture of macromonomers and/or commercial biocompatible monomers is polymerized, e.g. in a starved, miniemulsion or emulsion radical polymerization in water in the presence of a surfactant to the nanoparticle without necessitating additional subsequent steps for the actual production of the nanoparticles. The invention furthermore pertains to correspondingly made nanoparticles and uses thereof.

## Description

### TECHNICAL FIELD

The present invention relates to methods for the production of biodegradable nanoparticles, to biodegradable nanoparticles and to uses of such biodegradable nanoparticles, e.g. as drug carrier particles for medical uses.

### PRIOR ART

In the last decades nanoparticles have attracted a continuing interest, in particular for their use in biological application such as drug delivery, imaging, etc.

Despite the huge number of materials that can be used to produce nanoparticles for biological purposes, the most adopted are usually biodegradable polyesters, mainly polylactic acid (PLA), poly-glycolic acid (PGA), poly-caprolactone (PCL) and their copolymers (such as poly lactic-glycolic acid, PLGA). These polyesters are synthesized through ring opening polymerization (ROP) starting from cyclic monomers (lactide for PLA, glycolide for PGA, caprolactone for PCL, etc...) usually in bulk or in organic solution adopting an alcohol as initiator.

These polymers have to be dissolved in a suitable solvent and nanoparticles are then produced following different strategies, including both precipitation and emulsification followed by the solvent removal.

All the existing routes for the preparation of biodegradable nanoparticles lead to the production of nanoparticles with diameters under mild condition ranging from 100 to 200 nm. On the other hand, following radical polymerization processes, it is possible to obtain small nanoparticles (until 15 nm) in mild conditions and avoiding the use of organic solvents. The main problem in using such nanoparticles for biomedical applications is that they can be biocompatible (e.g. poly(methyl methacrylate)), but they are never biodegradable.

In respect of the relevant background state-of-the-art reference is made to the following publications:
G. He, Q. Pan, G. L. Rempel. "Synthesis of Poly(methyl methacrylate) Nanosize Particles by Differential Microemulsion Polymerization", Macromol. Rapid Commun., 24, 585-588 (2003). In this document Poly(methyl methacrylate) nanosize particles were synthesized by a differential microemulsion polymerization process. A particle size of less than 20 nm in diameter has been achieved with much milder conditions than those previously reported in the literature. Only the use of simple monomers as the starting material for the microemulsion polymerization is proposed.
A. Cretu, M. Kipping, et al., "Synthesis and characterization of hydrogels containing biodegradable polymers", Polym Int 57, 905-911 (2008). In this work the graft copolymers were synthesized using the macromonomer technique. In a first step, methacryloyl-terminated poly(L-lactide)macromonomers were synthesized in a wide molecular weight range using different catalysts. Subsequently, these macromonomers were copolymerized with 2-hydroxyethyl methacrylate in order to obtain a graft copolymer. These new materials resemble hydrogel scaffolds with a biodegradable component. The biodegradation was studied in hydrolytic and enzymatic environments. The influence of different parameters (molecular weight, crystallinity, ratio between hydrophilic and hydrophobic components) on the degradation rate was investigated. No nanoparticles are produced or proposed, and the polymerization process is a copolymerisation process.
A. Cretu, R. Gattin, et al., "Synthesis and degradation of poly (2-hydroxyethyl methacrylate)- graft-poly (e- caprolactone) copolymers" Polym Degr Stab 83 399-404 (2004). In this work poly (ε-caprolactone) macromonomers carrying a methacryloyl end groups were synthesized using different lanthanide derivatives as catalysts, and characterized by SEC and 1H NMR. Hydrophilic-hydrophobic copolymers from macromonomers and 2-hydroxyethyl methacrylate (HEMA) were obtained by solution free radical polymerization.
CF van Nostrum, et al., "Tuning the degradation rate of Poly(2-hydroxypropyl methacrylamide)-graft-oligo(lactic acid) Stereocomplex hydrogels", Macromol, 37, 2113-2118 (2004). In this work hydrogels based on poly(2-hydroxypropyl methacrylamide) with oligo(lactic acid) side chains are synthesized. The polymers were prepared by free radical copolymerization with 2-hydroxypropyl methacrylamide, so again in a copolymerisation process and without reference to nanoparticles.

The main idea of synthesizing very small nanoparticles through a starved radical polymerization is reported in He et al and in the references reported on the same paper. So far no evidences in using this process to synthesize biodegradable nanoparticles are reported in literature (both scientific and patent). Synthesis of macromonomers constituted by biodegradable side chains and hydrophilic backbone are widely reported in literature as for example given above. These macromonomers are used to synthesize hydrogels (so no nanoparticles) and usually the polymers produced present relatively high molecular weights.

### SUMMARY OF THE INVENTION

The present invention relates to the synthesis of macro-monomers with controlled molecular structure via ROP and production of hydrophilic-graft-biodegradable polymers for nanoparticles synthesis through radical polymerization. The proposed method encompasses two different steps. In the first step new macromonomers are synthesized through Ring Opening Polymerization (ROP), while in the second step the macromonomers are used to produce biocompatible and biodegradable nanoparticles via radical polymerization. The macromonomers are produced through ROP using a hydrophilic acrylate or methacrylate monomer bearing a suitable chemical group (e.g. OH) as initiator. Through the living polymerization mechanism of ROP, a controlled number of units of cyclic esters such as Lactide (LA), Glycolide (GL), Caprolactone (CL), etc. can be added to the acrylic initiator. The so-obtained macromonomers retain a reactive double bond from the acrylic moiety, which allows them to be polymerized through free-radical polymerization to synthesize nanoparticles. Due to the low viscosity of the macromonomers (which can be controlled by the number of cyclic ester units added to the acrylate initiator), very high conversion to polymer (up to 99%) is reached during radical polymerization. The produced polymers possess a comb-like structure, with a hydrophilic backbone of polyacrylate or methacrylate, grafted with oligo-lactides, or -glycolide, or - caprolacton, etc., which render the final polymer hydrophobic. Since oligo-lactides (and of course glycolide, caprolactone, and all the copolymers formed by these cyclic monomers) are biodegradable, nanoparticles made of these polymers will in the biodegradation process completely dissolve in water, leaving free biocompatible lactic acid and poly-hydrophilic acrylate or methacrylate.

So under the expression biodegradable in the context of this invention it is to be understood that the basic material comprises a hydrophilic backbone structure which is water-soluble and can e.g. in an organism be transported away (this backbone structure is not necessarily degraded, i.e. split up into individual blocks, in the biodegradation process) as well as graft side chains which are hydrophobic and which in the biodegradation process are indeed split into the (monomeric) building blocks. So the actual biodegradation process is a process in which the side chains are degraded into small transportable units until the backbone is essentially freed from the side chains, such that the hydrophilic backbone becomes dissolved and can be carried away. The degradation properties (speed, also as a function of different environments etc) can thereby be tailored by choosing the chemistry of the side chains, and in particular by choosing the length, i.e. the number of monomeric units, present for each side chain. The degradation properties can e.g. be adjusted such that in a corresponding biological environment the nanoparticles are essentially fully degraded within less than a year, less than six months, or even less than one month. It is however also possible to adjust the degradability such that the nanoparticles are essentially fully degraded after a week or even after a few days, e.g. 2-3 days.

The proposed process however not only allows this tailoring of the degradation properties, it also allows the production of nanoparticles directly with the actual production of a polymer material in the polymerization process of a subsequent second step. Furthermore it allows the incorporation of pharmaceutically active compounds, either introduced in this second step or after.

According to a first preferred embodiment of the proposed process, the polymerization in the second step is a starved or batch homo- or copolymerization in water in the presence of a surfactant to the nanoparticle. Preferentially can be a radical, e.g. a free radical, homo- or copolymerization process.

According to yet another preferred embodiment of the invention, the polymerization in the second step is a batch or starved polymerization process to form the nanoparticles, which is selected from the following group: emulsion polymerization; miniemulsion polymerization; nanoemulsion polymerization; suspension polymerization, preferably followed by nanoprecipitation or emulsion; solution polymerization, preferably followed by nanoprecipitation or emulsion; emulsion free-radical polymerization. Also combinations of these processes are possible.

In the second step a single macromonomer type as generated in the first step can be reacted, it is however also possible to polymerize a mixture of different macromonomers and/or a mixture of a macromonomer and at least one further (commercial conventional, preferably biocompatible) monomer, e.g., an acrylate monomer, in such a reaction, which then is not a homopolymerization but a copolymerization.

So one of the main novelties of this invention is to synthesize macromonomers that can undergo free-radical polymerization and, at the same time, can degrade under biological conditions into biocompatible compounds. In order to achieve these properties, hydrophilic unsaturated monomers are adopted (see Table 1) as initiators for an ROP process in order to produce oligoesters using different possible cyclic esters (see Table 2). In a typical experiment, the hydrophilic monomer is loaded into the reactor together with the cyclic monomer. The system is heated up until the melting point of the cyclic monomer (above 110 °C) is reached, and by using a suitable catalyst the reaction is carried out leading to the production of a biodegradable macromonomer. By tuning the ratio between initiator (alkene monomer) and cyclic ester, and the ratio between cyclic ester and catalyst, the molecular weight and hydrophobicity of the produced macromonomers can be easily tuned. As a result, macromonomers can be formed by one molecule of alkene and different units of ester group ranging from 1 to the desired value. In this invention we produced oligomer with ester group from 1 to 20. Once these macromonomers are synthesized they can be directly used as starting materials for radical polymerization. Through this process, the macromonomer, or a mixture of macromonomers, or a mixture of macromonomers and commercial monomers is added in different ways into water solution containing a suitable surfactant and a radical initiator. The system is heated up (from room temperature until 90 °C, as a result of the adopted initiator) and a starved emulsion, or miniemulsion, or emulsion free-radical polymerization is carried out. The composition of the monomeric phase can be changed in a step wise manner, leading to core-shell or multi layers morphology, or in a continuous manner, leading to a gradient in composition inside the particles. At the end of the reaction, it is possible to obtain a latex of small nanoparticles (the dimension is typically given by the radical polymerization process adopted, as well as by the amount of initiator and surfactant adopted) formed by polymer chains containing biodegradable oligo-esters grafted to hydrophilic polymer chains. Experiments on the degradation of these nanoparticles have also been performed. In particular the obtained latex has been maintained at a fixed temperature (i.e. 50°C) and the degradation process was followed adopting different analytical techniques. As a result it has been possible to prove that all the nanoparticles completely dissolve after a certain time leading to the release of the monomeric ester compounds and a water soluble polymer chain.

**Table 1. Examples ofhydrophilic monomers**

| **Hydrophilic monomer** | **Molecular structure** |
|---|---|
| 2-hydroxyethyl methacrylate, HEMA | 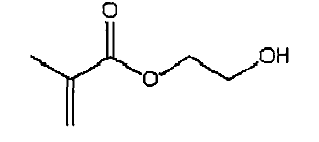 |
| 2-hydroxyethyl acrylate | 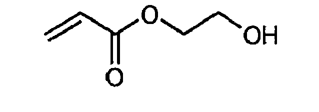 |
| N-(2-Hydroxypropyl)methacrylamide | 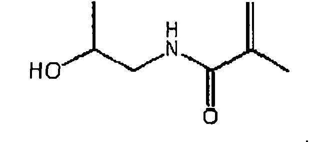 |
| N-(2-Hydroxyethyl)acrylamide, HEAA | 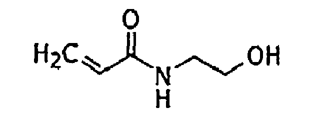 |
| N-(2-Hydroxyethyl)methacrylamide | 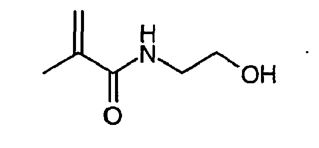 |
| Poly(ethylene glycol) ethyl ether methacrylate (this can be copolymerized, but normally not used as initiator for ROP) | 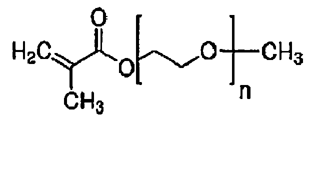 |
| *N*-[Tris(hydroxymethyl) methyl]acrylamide | 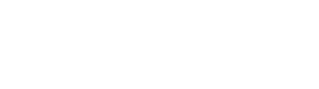 |
| 3-(Acryloyloxy)-2-hydroxypropyl - methacrylate | 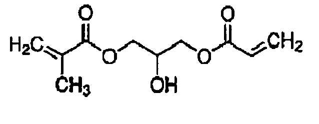 |
| Glycerol 1,3-diglycerolate diacrylate | 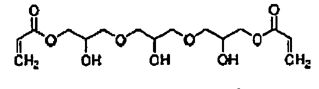 |
| 1,6-Hexanediylbis[oxy(2-hydroxy-3,1-propanediyl)] bisacrylate | 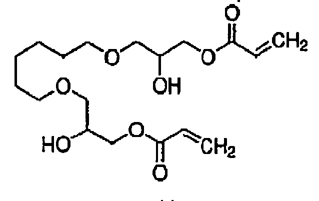 |
| *N*-(Hydroxymethyl)acrylamide solution | 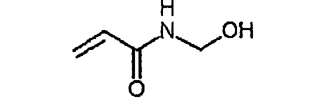 |
| 3-Chloro-2-hydroxypropyl methacrylate | 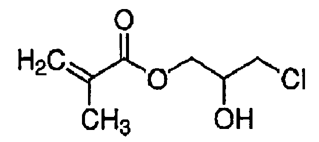 |

**Table 2. Examples of cyclic compounds that can be polymerized through ROP adopting the hydrophilic monomer as initiator.**

| **Cyclic monomer (suitable for ROP)** | **Molecular structure** |
|---|---|
| Glycolide | 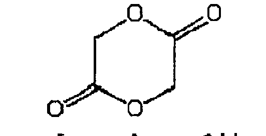 |
| Lactide | 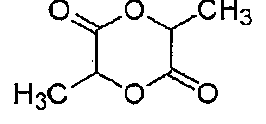 |
| Caprolactone | 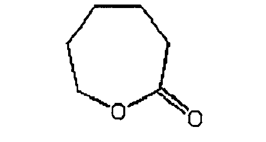 |
| Dioxanone | 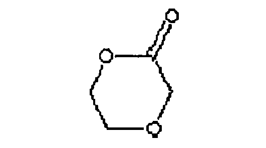 |
| Trimethylene carbonate | 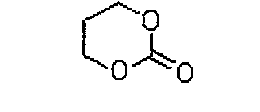 |
| 1,5-dioxepan-2-one | 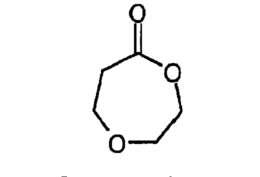 |
| Delta-valerolactone | 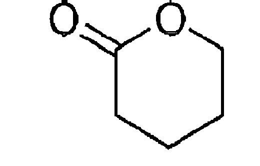 |
| Gamma-valerolactone | 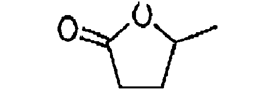 |
| Ethyl Ethylene Phosphate | 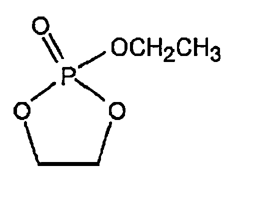 |
| ε-Caprolactam | 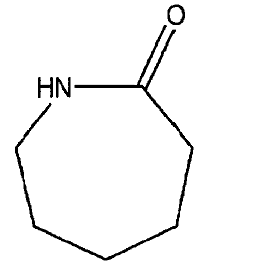 |

So the possibility to synthesize very small nanoparticles (having a diameter preferably smaller than 100 nm) through a starved free radical polymerization of macromonomers produced through ROP is introduced. The synthesis of similar macromonomers is reported in literature, even though their molecular weights are generally higher than those reported in this invention. Moreover no evidence of the use of these macromonomers in starved emulsion, miniemulsion, or emulsion polymerization has been reported. One of the main goals in using these low molecular weight oligomers is the possibility to achieve a very high conversion of the macromonomer used, thus minimizing toxicity effects. Moreover similar macromonomers have been already used to synthesize bulk hydrogels, while in this invention the process used permits the production of nanoparticles of hydrophobic homo-or copolymers through free radical polymerization. An important aspect of this invention is the choice of the surfactant used for the preparation of the nanoparticles. Since ionic surfactants are much more effective for the preparation of small nanoparticles, we have used biocompatible ionic surfactants, but also an ionic non-biocompatible surfactant (e.g. SDS), either alone or mixed with a non-ionic biocompatible surfactant (e.g. a polysorbate such as Tween 80), followed preferably by removal of SDS from the final product using ion-exchange resins. Both the removal of SDS and its replacement with a biocompatible surfactant and in particular the possibility to use in one step both the SDS and Tween 80 have never been published in the literature. In particular the latter permits to obtain a monodisperse distribution of nanoparticle sizes never found in literature.

More generally, the present invention relates to a method for the production of biodegradable nanoparticles with an average particle size of less than 400 nm, wherein
in a first step a macromonomer is prepared in a ring opening polymerization process between a hydrophilic acrylate compound (A) as an initiator and hydrophobic cyclic monomers (B), wherein the macromonomer comprises at least two repetitive units based on the cyclic monomer, and wherein
in a second step this macromonomer is polymerized to form the nanoparticles directly or indirectly (i.e. in a subsequent step), e.g. in a starved radical homo- or copolymerization in water in the presence of a surfactant to the nanoparticle.

According to a first preferred embodiment of the invention the hydrophilic acrylate compound (A) is selected from the group of 2-hydroxyethyl methacrylate, 2-hydroxyethyl acrylate, N-(2-hydroxypropyl)methacrylamide, N-(2-hydroxyethyl)acrylamide, N-(2-hydroxyethyl)methacrylamide, 2-aminoethyl methacrylate, 2-aminoethyl acrylate, glycerol monomethyl methacrylate, gylcerol monomethyl acrylate, Poly(ethylene glycol) ethyl ether methacrylate , or a mixture thereof wherein it is preferably a methacrylate compound, most preferably 2-hydroxyethyl methacrylate.

These acrylates can be substituted in general with an alkene bearing an OH or NH₂ or NH or SH group.

Preferentially, the hydrophobic cyclic monomer (B) is selected from the group of substituted or unsubstituted glycolide, lactide, substituted or unsubstituted caprolactone, substituted or unsubstituted dioxanone, substituted or unsubstituted trimethylene carbonate, 1,5-dioxepan-2-one or a mixture thereof, wherein it is preferably lactide.

According to a preferred embodiment, the number (n) of repetitive units based on the hydrophobic cyclic monomer in the macromonomer is in the range of 2-20, preferably in the range of 3-15.

Preferably, in the first step a catalyst, preferably an organo metal catalyst, more preferably an organotin, catalyst, most preferably Sn(Oct)₂, is used, and wherein the reaction temperature is kept at or above the melting point of the hydrophobic cyclic monomers (B), prcfcrably at a temperature of at least 100°C, more preferably at a temperature of at least 110°C. The same reaction can be carried out in an organic solvent at a lower temperature (>50-60°C).

Based on the ratio between the hydrophilic acrylate compounds (A) and the hydrophobic cyclic monomer (B) the molecular weight of the produced macromonomers can be tuned to be in the range of 200-1500 g/mol, preferably in the range of 300-1100 g/mol.

Further preferably the macromonomer (or a mixture of different macromonomers, or of a macromonomer and other commercially available, preferably biocompatible monomers, preferably acrylate monomers) which is the starting material for the second step comprises only one hydrophilic acrylate compound element per molecule.

According to yet another preferred embodiment, the nanoparticles have an average particle size of less than 300 nm, preferably of less than 150nm or 100nm, preferably less than 50 nm, or even less than 40 nm, typically in the range of 25-60 nm. The nanoparticles can either have a homogeneous morphology, or core-shell morphology, in which the composition of the core differs from that of the shell, or a multilayers morphology, with a series of layers having each a different composition from the others, and/or gradient in composition, which changes essentially continuously while moving from the center to the outer rim of the nanoparticles.

The surfactant used in the second step is preferably a ionic and/or non-ionic surfactant, wherein preferably the ionic surfactant is selected to be SDS, and wherein preferably the non-ionic surfactant is selected to be a sorbitan ester, preferably selected from Span 20 (Sorbitan monolaurate), Span 40 (Sorbitan monopalmitate), Span 60 (Sorbitan monostearate), Span 65 (Sorbitan tristearate), Span 80 (sorbitan monooleate), or from polyoxyethylene sorbitan esters such as the Tween compounds, i.e. Tween 80 (polyoxyethylene sorbitan monooleate), Tween 20 (polyoxyethylene sorbitan monolaurate), Tween 40 (polyoxyethylene sorbitan monopalmitate), Tween 60 (polyoxycthylcnc sorbitan monostearate), more preferably polysorbate 80 (other steric surfactants can also be selected or mixtures of such systems), and wherein preferably after the second step any ionic surfactant used is removed, preferably using ion exchange chromatography. Preferentially a mixture of an ionic and of a non-ionic surfactant is used, preferably in a mass ratio of 2:1 to 1:2.

According to a further preferred embodiment, the second step the molecular weight of the resulting homo- or copolymer is adjusted to be in the range of 5000-20,000 g/mol, preferably in the range of 8000-15,000 g/mol.

In the second step preferably a radical initiator, preferably selected from a persulfate compound (or another free-radical initiator), more preferably selected from potassium persulphate, is used and/or wherein in the second step further functional compounds, in particular pharmaceutically active compounds are present to be embedded in and/or attached to the nanoparticles.

In the second step an organic solvent, preferably CH₂Cl₂ or a similar organic solvent can be added to the reaction in a sufficient amount to diminish the viscosity in particular if the number (n) of the repetitive units of the macro monomer is larger than 5.

Furthermore the present invention relates to a nanoparticle obtainable or obtained using a method as given above, preferably with an average particle size of less than 100 nm, more preferably of less than 50 nm.

Such a nanoparticle preferentially comprises at least one pharmaceutically active compound in a pharmaceutically active amount for controlled release so as a drug delivery vehicle.

In addition to that, the present invention relates to the use of a nanoparticle according to the above description as a drug delivery carrier.

Further embodiments of the invention are laid down in the dependent claims.

### BRIEF DESCRIPTION OF THE FIGURES

Figures 1a-e show the degradation behavior of nanoparticles produced with the macromonomer characterized by n equal to 3.
Figures 2a-d show the degradation behavior of nanoparticles produced with the macromonomer characterized by n equal to 8.

### DESCRIPTION OF PREFERRED EMBODIMENTS

Preferred embodiments of the invention are described in the following, wherein the description is for the purpose of illustrating the present preferred embodiments of the invention and not for the purpose of limiting the same.

### Synthesis of macromonomers:

### Reactants:

- 2-hydroxyethyl methacrylate (ROP initiator)
- Lactide (cyclic monomer)
- Sn(Oct)₂ (FDA approved catalyst for ROP)

The bulk reaction is carried out at 130 °C. At this temperature the solution is homogeneous. By varying the ratio between lactide and HEMA it is possible to produce oligomers with different lactic acid chain lengths as reported in the reaction scheme 1:

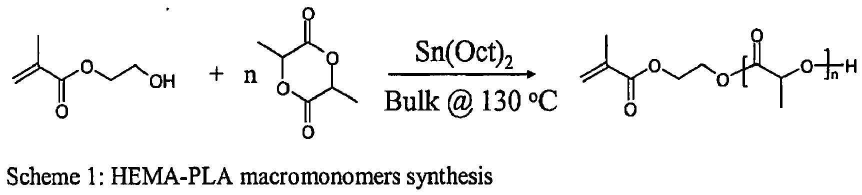

In Table A both the molecular weight (MW) of synthesized macromonomer and the relative degree of polymerization (n) determined through NMR analysis are reported. Since the aim of this invention is to produce materials suitable for bio-application, the most interesting macromonomers are those characterized by a low MW. These compounds in fact, present a higher conversion in radical polymerization because of their limited steric hindrance and lower viscosity compared to the higher MW macromonomers. As a result they lead to the formation of polymers without residual monomers (or in any case with a negligibly low concentration ofunreacted monomers), which are potentially toxic.

**Table A (MW in g/mol)**

| # | MW | n |
|---|---|---|
| 1 | 275.15 | 2.016 |
| 2 | 344.45 | 2.98 |
| 3 | 403.6 | 3.8 |
| 4 | 479.92 | 4.86 |
| 5 | 539.25 | 5.684 |
| 6 | 597 | 6.49 |
| 7 | 614 | 6.7 |
| 8 | 748.48 | 8.59 |
| 9 | 816.88 | 9.54 |
| 10 | 892 | 10.58 |
| 11 | 1058 | 12.89 |

As the data reported in Table A indicate, through ROP it is possible to obtain all the desired oligomers. Moreover NMR analysis shows that the reaction reported in scheme 1 is complete and leads to the chemical structure reported on the same figure, on the right.

### Synthesis of nanoparticles

To synthesize the biodegradable nanoparticles (NPs), a starved polymerization process has been adopted. Nanoparticle are formed by poly-(2-hydroxyethyl methacrylate)-graft-oligo(lactic acid) polymer chains as reported in scheme 2:

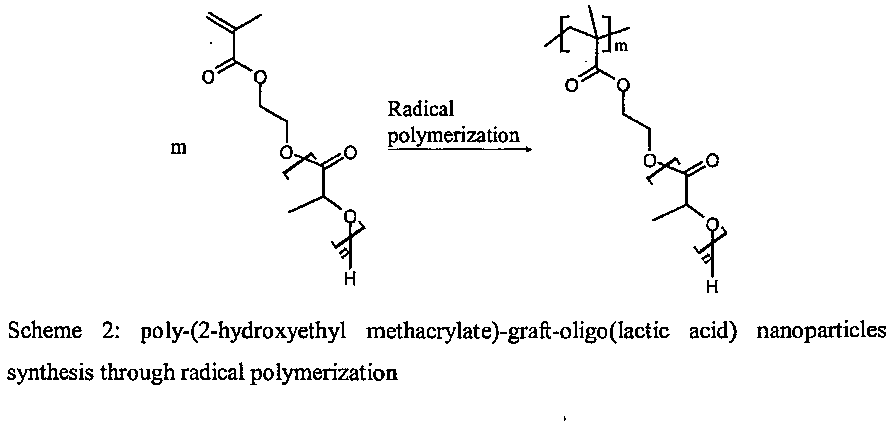

### Standard recipe:

| | | |
|---|---|---|
| - | Water | 100 ml |
| - | Macromonomer | 5 grams |
| - | Surfactant (SDS, Tween 80 or a mixture thereof) | 1 gram |
| - | Radical initiator: KPS (potassium persulfate) | 0.08 grams |
| - | Reaction temperature | 70/80 °C |
| - | Injection rate (using a syringe pump) | 6 ml/h |
| - | CH₂Cl₂ can be used to diminish the viscosity of higher MW oligomers (when n≤6), in order to be able to use the same syringe pump adopted for low MW oligomers. | |

Water and surfactant are placed in a 250 ml reactor and heated up until the reaction temperature (70 or 80 °C). The system is first stripped using nitrogen to avoid the presence of oxygen, which strongly inhibits active radicals, and then the initiator is added. Subsequently, the macromonomer is slowly injected into the reactor through a syringe pump. After the injection of the whole amount of macromonomer, the system is kept at the reaction temperature for one hour and a half to achieve full conversion. The obtained nanoparticles are then characterized in terms of polymer molecular weight (by GPC) and size (by Light Scattering, LS).

In order to check the surfactant effect on the produced nanoparticles we used three different recipes:
1. loading 1g of SDS
2. loading 1 g of Tween 80
3. loading 1 g of SDS plus 1 g of Tween 80

Since the particles are produced for bio-applications, approved compounds have to be adopted. Since SDS is not allowed to be used for biological purposes, it has to be removed from the final products and replaced with biocompatible surfactants (e.g. Tween 80). On the other hand, SDS is one of the most efficient surfactant that can be used for the production of small particles (see Table B). Therefore, the following strategy is adopted to replace SDS in both recipe 1 and 3 previously reported. A sufficient amount of Ion Exchange resins (IEX) are added to the particle dispersion in order to completely remove the SDS. Then, for products obtained through recipe 1, 1 gram of Tween 80 is added in order to maintain stable the latex (which, without surfactant is unstable and tends to aggregate). In Table B nanoparticle dimensions obtained with different macromonomer are reported as a function of the adopted recipe (number average molecular weight is around 10000 Da for all the samples).

**Table B (Nanoparticles diameter in nm)**

| n | SDS | Tween 80 | SDS + Tween 80 |
|---|---|---|---|
| 1 | - | - | - |
| 2 | 44 | 155 | 56 |
| 3 | 31 | 240 | 61 |
| 4 | 34 | 147 | 54 |
| 5 | 31 | - | 61 |
| 6 | 27 | 230 | 41 |
| 7 | 25 | 196 | 43 |
| 8 | 25 | - | 42 |
| 9 | 24 | - | 44 |
| 10 | 27 | - | 43 |

In all the samples, small quantity of insoluble white precipitate is found. This co-product is formed by cross-linked polymer matrices obtained through side reactions of the free hydroxyl group of the macromonomer. This side reactions are particularly active for the macromonomer with n=1 (that presents the higher number of free OH groups per unit of reacting macromonomer) for which the amount of crosslinked polymers is not negligible. To avoid these side reactions the reaction temperature has to be kept as lower as possible. As a result reactions are then performed at 70°C with the same initiator, but different initiation mechanisms can be utilized that allow one to decrease the polymerization temperature down to 25°C, if necessary.

Clearly, the direct use of Tween 80 leads to the synthesis of larger nanoparticles (Tween 80 belongs to the steric-surfactant category). After the nanoparticles synthesis accordingly to recipe 1 and 3, the SDS has to be removed using IEX. Nanoparticles dimensions as long as diameter polidispersity (PD), after SDS removal, are reported in Table C.

**Table C (Nanoparticles diameter in nm)**

| n | SDS | PD | SDS + Tween 80 | PD |
|---|---|---|---|---|
| 1 | - | - | - | - |
| 2 | 50 | 0.19 | 56 | 0.07 |
| 3 | 50 | 0.28 | 62 | 0.04 |
| 4 | 36 | 0.10 | 56 | 0.01 |
| 5 | 32 | 0.10 | 61 | 0.02 |
| 6 | 34 | 0.12 | 44 | 0.06 |
| 7 | 29 | 0.18 | 43 | 0.01 |
| 8 | 27 | 0.11 | 42 | 0.04 |
| 9 | 26 | 0.14 | 45 | 0.06 |
| 10 | 29 | 0.15 | 44 | 0.06 |

It is observed that, by using SDS and then replacing it with Tween 80, the size of the particles reaches the smallest values, while the use of SDS+Tween 80 followed by removal of SDS leads to the synthesis of larger particles. On the other hand, the latter synthesis leads to a much narrower nanoparticle distributions.

Finally, it is important to notice that a complete monomer conversion is found for all the adopted macromonomer.

### Degradation of nanoparticles

In order to demonstrate the possibility of these nanoparticles to be completely degraded into non toxic and biocompatible compounds, a series of degradation experiments have been performed.

Standard procedure:
- Latex produced with a macromonomer and stabilized with SDS is placed in a vial.
- Latex is kept at constant temperature (50 °C)
- After a certain time, latex samples are analyzed in order to check the degradation behavior.

In order to analyze the nanoparticles degradation different techniques are used. First of all, the solution pH is measured as a function of the degradation time in order to qualitatively demonstrate that the polymer is degrading, since lactic acid is released during as a result of the degradation and a pH decrease is expected. In parallel the nanoparticle diameter is measured through LS. At given time intervals, sample aliquots are collected to carry out quantitative analyses. In particular, nanoparticles are destabilized and precipitated out of the solution by adding concentrated NaCl solution to the sample under investigation, a procedure that requires the use of a latex stabilized by an ionic surfactant, such as SDS. With a steric surfactant, the recovery of the nanoparticles would be more problematic. The precipitate is separated by centrifugation and analyzed by GPC, while the supernatant is analyzed by HPLC in order to check the presence of chemical species coming from the degradation process.

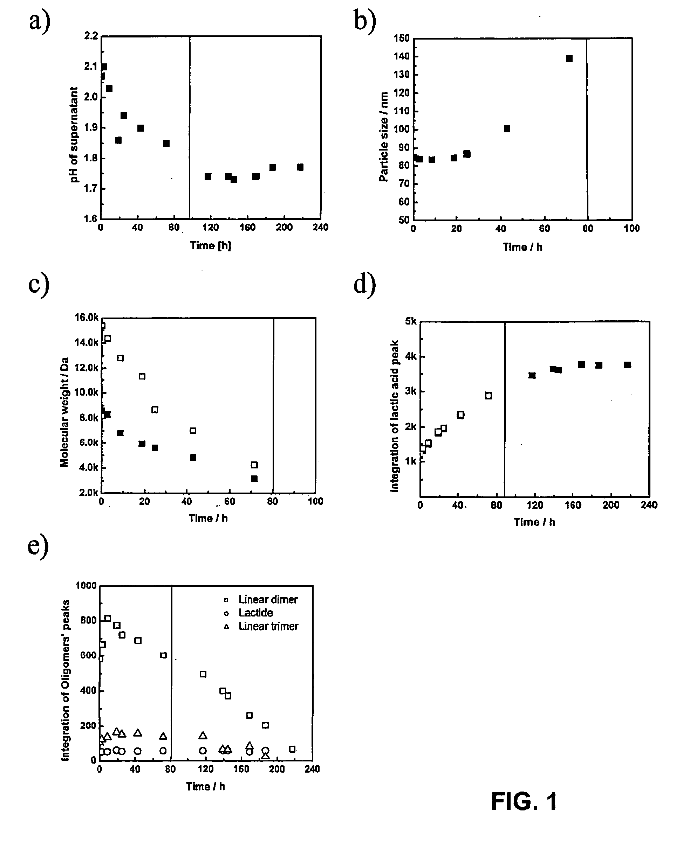

In Figure 1a-e the degradation behavior of the nanoparticles produced adopting the macromonomer with n=3 is reported. In particular the pH of the supernatant (1a), the particles size (1b), the polymer molecular weight (number average MW and weight average MW) (1c), the relative lactic acid amount in the supernatant (obtained integrating the NMR spectra) (1d) and the oligomers composition of the supernatant (1e) as a function of the degradation time are reported.

All reported data clearly confirm the degradation of the produced nanoparticles. In all the figures a vertical solid line indicates the disappearance of the dispersion turbidity. Since the dispersion turbidity is related to the presence of nanoparticles, a decrease of the turbidity to zero indicates complete degradation of the nanoparticles. In particular:
- Figure 1a shows a decrease of the pH as expected.
- Figure 1b shows an increase of the nanoparticle size as expected. In fact during the degradation of the PLA side chain, the hydrophilicity of the polymer increases, leading to a nanoparticles swelling that results into an increase of the NP size. Moreover, after the turbidity disappearance (80 h approx.) no more nanoparticles are detected.
- Figure 1c shows a decrease of both the number average MW and weight average MW during the time, as expected. In particular it is worth noting how the MWs of water soluble polymers determined after the nanoparticle disappearance reach the value calculated for the PHEMA without any PLA branch. This is an additional confirmation of the complete degradation of all the PLA chains.
- Figure 1d shows an increase of the lactic acid release as expected. Two different slopes can be clearly recognized before and after the NP disappearance.
- Figure 1e shows the concentration profile of oligomers released in the supernatant. Since these PLA oligomers are water soluble, their degradation still continues after the nanoparticles disappearance and leads to a slightly increase in the lactic acid concentration, as Figure 1d shows (after the vertical solid line)

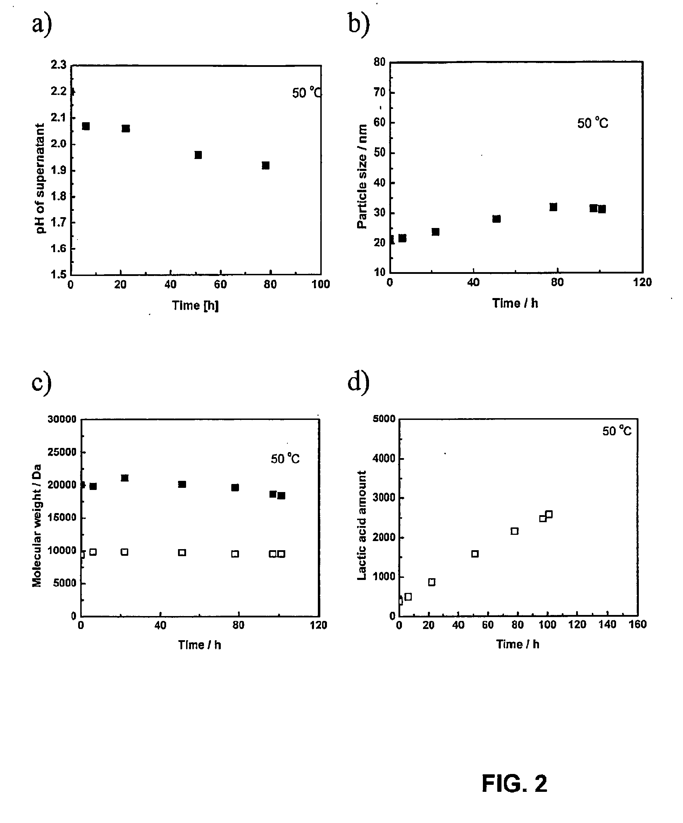

The same experiments carried out with the macromonomer having n equal to 3 are performed with a macromonomer with a larger number of lactide units added (n equal to 8), in order to demonstrate the capability of tuning the degradation behavior of the produced nanoparticles by modifying the chain length of the macromonomers. The obtained results are reported in Figure 2a-d.

By analyzing data reported in figure 2 it is possible to conclude that the same degradation pattern is found for the larger macromonomer. However, the degradation requires a longer time to reach completion, as a result of the higher MW of the PLA chain. These preliminary results clearly demonstrate the possibility to tune the degradation behavior by changing the macromonomer adopted for the NP synthesis.

The feasibility in extending the reported macro-monomers synthesis to produce hydrophilic-graft-biodegradable polymers via ROP is confirmed by the production of caprolactone-based macromonomers.

### Synthesis of macromonomers:

Macromonomers are synthesized as previously reported; in this case caprolactone is used in place of lactide. In Table D the molecular weight of produced macro-monomers determined by NMR and the relative degree of polymerization are reported.

**Table B (MW in g/mol)**

| # | MW | n |
|---|---|---|
| 12 | 245.50 | 1.005 |
| 13 | 359.10 | 2.006 |
| 14 | 473.24 | 3.008 |
| 15 | 586.24 | 3.996 |
| 16 | 699.81 | 4.991 |
| 17 | 1040.98 | 7.980 |

Synthesized caprolactone-based macromonomers are adopted to prepare nanoparticles suitable for bio-application. As reported for the lactide-based macromonomers, SDS, Tween 80 and SDS plus Tween 80 are adopted as surfactants. In Table E nanoparticle dimensions obtained with different macromonomers are reported.

**Table E (Nanoparticles diameter in nm)**

| # | SDS | Tween 80 | SDS + Tween 80 |
|---|---|---|---|
| 12 | 67 | 180 | 81 |
| 13 | 72 | 165 | 77 |
| 14 | 70 | 170 | 84 |
| 15 | 68 | 182 | 79 |
| 16 | 77 | 188 | 83 |
| 17 | 75 | 173 | 88 |

### Copolymerization

The synthesized macromonomers can be adopted as starting material for a copolymerization processes. In particular both lactide-based and caprolactone-based macromonomers are copolymerized with pegylated HEMA. This copolymerization allows to obtain in one step pegylated nanoparticles without the use of a surfactant:

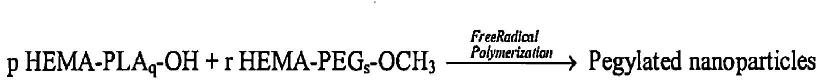

Pegylated nanoparticles are of importance in order to synthesize long-term circulating stealth nanoparticles. Moreover the possibility to obtain through a free radical polymerization process nanoparticles without the use of any surfactant (the particles are self stabilized by the presence of the PEG chain) is of great interest since no further chemicals have to be adopted.

### Synthesis of pegylated nanoparticles:

### Standard recipe:

| | | |
|---|---|---|
| - | Water | 100 ml |
| - | macromonomer (HEMA-PLA/CL) | 3 grams |
| - | pegylated HEMA | from 0.3 to 3 grams |
| - | radical initiator (KPS) | 0.08 grams |
| - | reaction temperature | 70°C |
| - | injection rate | 6 ml/h |

The procedure adopted to synthesize pegylated nanoparticles is the same as previously reported. In Table F nanoparticle dimensions obtained with different co-monomer ratio are reported.

| # | p (grams) | q | R (grams) | s | Diameter (nm) |
|---|---|---|---|---|---|
| 18 | 3.0 | 5 | 1.0 | 5 | 64 |
| 19 | 3.0 | 5 | 1.0 | 7 | 58 |
| 20 | 3.0 | 5 | 1.0 | 23 | 65 |
| 21 | 3.0 | 5 | 1.0 | 45 | 88 |
| 22 | 3.0 | 5 | 1.0 | 90 | 106 |
| 23 | 3.0 | 8 | 1.0 | 45 | 91 |
| 24 | 3.0 | 5 | 0.3 | 45 | - |
| 25 | 3.0 | 5 | 0.7 | 45 | 280 |
| 26 | 3.0 | 5 | 3.0 | 45 | 45 |

As it can be observed, except for low pegylated HEMA content, the copolymerization of macromonomers and pegylated HEMA leads to nanoparticles even without the use of any surfactant. In particular nanoparticles produced show an increase of the diameter for lower pegylate HEMA concentration and larger pegylated HEMA molecular weight.

## Claims

1. Method for the production of biodegradable nanoparticles with an average particle size of less than 400 nm, wherein
in a first step a macromonomer is prepared in a ring opening polymerization process between a hydrophilic acrylate compound (A) as an initiator and hydrophobic cyclic monomers (B), wherein the macromonomer comprises at least two repetitive units based on the cyclic monomer, and wherein
in a second step this macromonomer is polymerized.

2. Method according to claim 1, wherein the polymerization in the second step is a starved or batch, preferably radical, homo- or copolymerization in water in the presence or in the absence of a surfactant to the nanoparticle.

3. Method according to claim 1 or 2, wherein the polymerization in the second step is a batch or starved polymerization process to form the nanoparticles selected from the group: emulsion polymerization; miniemulsion polymerization; nanoemulsion polymerization; suspension polymerization, preferably followed by nanoprecipitation or emulsion; solution polymerization, preferably followed by nanoprecipitation or emulsion; emulsion free-radical polymerization.

4. Method according to any of the preceding claims, wherein in the second step a mixture of different macromonomers and/or a mixture of at least one macromonomer and at least one further monomer, preferably a biocompatible monomer, further preferably a biocompatible acrylate monomer, is polymerized.

5. Method according to any of the preceding claims, wherein the hydrophilic acrylate compound (A) is selected from the group of 2-hydroxyethyl methacrylate, 2-hydroxyethyl acrylate, N-(2-hydroxypropyl)methacrylamide, N-(2-hydroxyethyl)acrylamide, N-(2-hydroxyethyl)methacrylamide, 2-aminoethyl methacrylate, 2-aminoethyl acrylate, glycerol monomethyl methacrylate, gylcerol monomethyl acrylate, Poly(ethylene glycol) ethyl ether methacrylate, or a mixture thereof wherein it is preferably a methacrylate compound, most preferably 2-hydroxyethyl methacrylate.

6. Method according to any of the preceding claims, wherein the hydrophobic cyclic monomer (B) is selected from the group of substituted or unsubstituted glycolide, lactide, substituted or unsubstituted caprolactone, substituted or unsubstituted dioxanone, substituted or unsubstituted trimethylene carbonate, 1,5-dioxepan-2-one or a mixture thereof, wherein it is preferably lactide.

7. Method according to any of the preceding claims, wherein the number (n) of repetitive units based on the hydrophobic cyclic monomer in the macromonomer is in the range of 2-20, preferably in the range of 3-15.

8. Method according to any of the preceding claims, wherein in the first step a catalyst, preferably an organo metal catalyst, more preferably an organo tin, catalyst, most preferably Sn(Oct)₂, is used, and wherein the reaction temperature is either kept at or above the melting point of the hydrophobic cyclic monomers (B), preferably at a temperature of at least 100°C, more preferably at a temperature of at least 110°C, or, if the reaction is carried out in an organic solvent at a temperature in the range of 40-70°C, preferably of 50-60°C
and/or
wherein in the second step a radical initiator, preferably selected from a persulfate compound, more preferably selected from potassium persulphate or another free radical initiator, is used and/or wherein in the second step further functional compounds, in particular pharmaceutically active compounds are present to be embedded in and/or attached to the nanoparticles.

9. Method according to any of the preceding claims, wherein based on the ratio between the hydrophilic acrylate compounds (A) and the hydrophobic cyclic monomer (B) the molecular weight of the produced macromonomers is tuned to be in the range of 200-1500 g/mol, preferably in the range of 300-1100 g/mol, and wherein preferably the macromonomer comprises only one hydrophilic acrylate compound element per molecule.

10. Method according to any of the preceding claims, wherein the nanoparticles have an average particle size of less than 300nm, preferably of less than 150nm, more preferably less than 100 or less than 50 nm, wherein the morphology of the nanoparticles is homogeneous, core shell, or multilayer or with an essentially continuous gradient in composition from the centre to the outer rim of the nanoparticles.

11. Method according to any of the preceding claims, wherein the surfactant in the second step is a ionic and/or non-ionic surfactant, wherein preferably the ionic surfactant is selected to be SDS, and wherein preferably the non-ionic surfactant is selected to be a sorbitan monolaurate, sorbitan monopalmitate, sorbitan monostearate, sorbitan tristearate, sorbitan monooleate, polyoxyethylene sorbitan monooleate, polyoxyethylene sorbitan monolaurate, polyoxyethylene sorbitan monopalmitate, polyoxyethylene sorbitan monostearate, more preferably polysorbate 80, and wherein preferably after the second step any ionic surfactant used is removed, preferably using ion exchange chromatography wherein preferably a mixture of an ionic and of a non-ionic surfactant is used, preferably in a mass ratio of 2:1 to 1:2.

12. Method according to any of the preceding claims, wherein in the second step the molecular weight of the resulting homopolymer or copolymer is adjusted to be in the range of 5000-20,000 g/mol, preferably in the range of 8000-15,000 g/mol and/or
wherein in the second step an organic solvent, preferably CH₂Cl₂ is added to the reaction in a sufficient amount to diminish the viscosity in particular if the number (n) of the repetitive units of the macro monomer is larger than 5.

13. Nanoparticle obtainable or obtained using a method according to any of the preceding claims, preferably with an average particle size of less than 300nm, preferably less than 150 nm, more preferably less than 100 nm, or of less than 50 nm.

14. Nanoparticle according to claim 13, wherein it comprises at least one pharmaceutically active compound in a pharmaceutically active amount for controlled release.

15. Use of a nanoparticle according to any of claims 13 or 14 as a drug delivery carrier.
